# EUROPEAN PATENT APPLICATION

(11) **EP 4 834 413 A2**
(43) Date of publication of application: **04.11.2026**
(21) Application number: 26205968.6
(22) Date of filing: 10.11.2021
(51) Int. Cl.: A61M 39/10

(54) **MEDICAL WASTE COLLECTION SYSTEM**

(30) Priority: 10.11.2020 US 202063111848 P
(62) Document number(s) of the earlier application(s) in accordance with Art. 76 EPC: 21819672.3 / 4 243 889
(71) Applicant: Stryker Corporation, Portage, MI 49002 (US)
(72) Inventor: ZOLLINGER, Michael, Chelsea, MI 48118 (US); LADUKE, Peter, Holland, MI 49424 (US); KIRSCHENSTEINER, Jeffrey, Kalamazoo, MI 49006 (US); EDINGER, Benjamin, Grand Haven, MI 49417 (US)
(74) Representative: Röthinger, Rainer

(57) **Abstract**

A medical waste collection system (20) for collecting medical waste material through a manifold (30) during a medical procedure comprises a waste container (24), a vacuum source configured to provide a vacuum on the waste container (24), and a receiver (26) coupled to the waste container (24). The receiver (26) comprises a housing (40) comprising an opening (28) into which the manifold (30) is configured to be inserted, the housing (40) further comprising a receiver outlet (36), an inlet mechanism (32) coupled to the housing (40) so as to be moveable in proximal and distal directions, wherein the inlet mechanism (32) comprises a suction inlet (33), and a suction outlet (34) in fluid communication with the suction inlet (33), a sled assembly (58) moveably coupled to the housing (40) and operably coupled to the inlet mechanism (32), wherein the sled assembly (58) is configured to be moved in a proximal direction during insertion of the manifold (30) into the receiver (26) in the proximal direction to facilitate the inlet mechanism (32) moving correspondingly in the distal direction to establish fluid communication between the suction outlet (34) and the receiver (26) outlet, and a motion conversion assembly (64) comprising a cam mechanism (96) operatively coupling the sled assembly (58) and the inlet mechanism (32) to facilitate the respective corresponding movements of the sled assembly (58) and the inlet mechanism (32) in the proximal and distal directions.

## Description

### RELATED APPLICATIONS

This application claims priority to and the benefit of U.S. Provisional Patent Application No. 63/111,848, filed on November 10, 2020, the entire contents of which are hereby incorporated by reference.

### BACKGROUND

A byproduct of some surgical procedures is the generation of liquid, semisolid, and/or solid waste material. The liquid waste material may include bodily fluids and irrigating solution(s) at the surgical site, and the solid and semisolid waste material may include bits of tissue and pieces of surgical material(s). The medical waste, regardless of its phase, is preferably collected so it neither fouls the surgical site nor becomes a biohazard in the medical suite in which the procedure is being performed.

The medical waste may be removed from the surgical site through a suction tube under the influence of a vacuum provided by a suction/vacuum source. One exemplary medical waste collection system is sold under the tradename NEPTUNE by Stryker Corporation (Kalamazoo, Mich.) with certain versions of the medical waste collection system disclosed in commonly-owned United States Patent Publication No. 2005/0171495, published August 4, 2005, International Patent Publication No. WO 2007/070570, published June 21, 2007, and International Patent Publication No. WO 2014/066337, published May 1, 2014, the entire contents of each are incorporated herein by reference.

The medical waste collection system may include a receiver to removably receive a manifold, and the manifold facilitates interfacing the suction tube with the medical waste collection system. The manifold may also include a filter element for filtering the waste material to avoid clogging or compromise of components of the medical waste collection system. Facilitating safe and efficient repeated coupling and decoupling of manifolds with the medical waste collection system requires that the receiver be robust, which remains an area of particular interest and development.

### SUMMARY

With the scope of the invention defined by the claims and clauses included herein without limiting effect of the Summary, the present disclosure is directed to a receiver for a medical waste collection system. The medical waste collection system includes at least one waste container defining a waste volume for collecting and storing the waste material, and the receiver coupled to the waste container with a conduit. A vacuum pump is supported on the cart and configured to draw suction on the waste container(s) and the receiver. The receiver includes an inlet mechanism that is movable to be coupled with a manifold to establish fluid communication between the manifold and the waste container in a manner to be further described. The inlet mechanism may include a suction inlet, and a suction outlet in fluid communication with the suction inlet. The suction inlet is configured to be arranged in fluid communication with the manifold, and the suction outlet is configured to be arranged in fluid communication with a receiver outlet. The receiver includes a housing defining an opening sized to removably receive a manifold.

The inlet lock assembly may include a latch pivotably coupled to a lower wall of the housing of the receiver. The inlet lock assembly may be biased to a locked position by a latch biasing member. The latch is pivotable about a latch axis and includes a head portion, and a tail portion positioned opposite the latch axis. The tail portion may be longer than the head portion. The latch biasing member may be a coil spring disposed between the head portion and the lower wall of the housing. A ramped surface of the spine of the manifold may directly contact the head portion at a deflection angle so as to cause pivoting of the latch about the latch axis. The base of the inlet mechanism may define a cavity for receiving the tail portion of the latch when the latch is in the unlocked position.

Claws may be coupled to the sled assembly. The housing may define one or more channels extending generally in proximal-to-distal directions on either side of the sled assembly. Each of the claws may include first and second claw pins that are slidable within each channel. The channels guide the path of the pins as the sled assembly moves proximally and distally. The first and second claw pins and channels cooperate to cause the claws to articulate inwardly toward the manifold responsive to movement of the sled assembly in the proximal direction.

The lock assembly may include an arm rotatably coupled to the housing, and an arm biasing member, such as a spring. The arm biasing member biases the arm to a locked configuration in which the arm is abuts the manifold. The arms of the lock assembly are pivotably coupled to the housing and positioned opposite a void space into which the manifold is to be situated. The arms are biased to the locked position with springs coupling the arms and the housing. The arms may each include a shoulder that is biased inwardly. The sled body has an arm retention surface configured to abut the shoulder of the arm of the lock assembly and retain the arm in an unlocked configuration. Interference between the distally-directed surfaces and the shoulders prevent distal movement of the manifold within the receiver.

The actuator may include a ramped surface that is configured to abut the arm and rotate the arm in opposition to the arm biasing member. The actuator itself may be biased to an outward distal position with a biasing element so as to be operated by a push input. The biasing element may be positioned between the actuator and the housing. The actuator and the biasing element may be slidably disposed on rails extending within the housing of the receiver in the proximal-to-distal direction. The proximal movement of the actuator moves ramped surfaces into engagement with fingers of the arms. The fingers and the shoulders of the arms are positioned opposite the pivot.

The motion conversion assembly may include a cam mechanism and a cam follower mechanism. The cam mechanism may include a cam body rotatably coupled to the housing about a cam center axis. The cam follower mechanism may include a lever rotatably coupled to the housing about a lever axis spaced from the cam center axis. The cam follower mechanism also includes a roller rotatably coupled to the lever and configured to be in direct rolling contact with the cam body. The motion conversion assembly is configured to convert movement of the sled assembly in the proximal direction into movement of the inlet mechanism in the distal direction during insertion of the manifold, and conversely convert movement of the sled assembly in the distal direction into movement of the inlet mechanism in the proximal direction during removal of the manifold.

The cam mechanism may include an inlet mechanism engagement pin extending from the cam body and radially spaced from the cam center axis. The inlet mechanism engagement pin may be received in an inlet slot and configured to move within the inlet slot and abut the inlet base to move the inlet mechanism proximally and distally in response to rotation of the cam body. The cam mechanism may include a sled engagement pin extending from the cam body and radially spaced from the cam center axis. The sled engagement pin may be received in a sled slot. The sled body may also have a proximal wall and a distal wall defining proximal and distal ends of the sled slot, respectively. The proximal and distal walls allow the sled body to continue to move proximally after the suction outlet of the inlet mechanism is aligned with the receiver outlet.

The distance between the slot engagement pin and the cam center axis may be greater than the distance between the inlet mechanism engagement pin and the cam center axis. The roller is rotatably coupled to a first end of the lever, and a second end of the lever is resiliently coupled to the housing with a lever biasing member. The lever axis may be spaced closer to the first or the second end of the lever. The roller is in direct contact with the cam, and the lever biasing member pivots the lever about the third pin. The cam includes an eccentric surface relative to the cam center axis. The roller engages the eccentric surface, and the relative distances of the certain points circumferentially arranged on the eccentric surface results in greater pivoting of the lever against the bias from the lever biasing member.

An electronics module may be coupled to an upper wall of the housing. The electronics module may include any number of electronic subcomponents, for example, sensors, integrated circuits, printed circuit boards, memory, communications means, and electrical or data ports. A detectable element may be positioned on the sled assembly. The initial return movement from the motion conversion assembly may be of sufficient magnitude to space apart the detectable element from the one or more sensors by a distance in which the one or more sensors generates a sled change signal. The sled change signal may be transmitted to the system processor, and any type of front-end functionality may be realized based on the sled change signal.

The suction outlet is in fluid communication with the receiver outlet and the conduit. The inlet mechanism is moveable proximally along an inlet axis disposed at a decline angle relative to a reference horizontal axis with respect to gravity. The conduit may include a receiver coupling portion extending along a conduit axis from the receiver toward the waste container. The conduit axis may be oblique to the inlet axis. The conduit axis may be disposed vertically with respect to gravity. The suction outlet may extend along a suction outlet axis that is oblique to the conduit axis. A seal may be coupled to the housing to cover the receiver outlet. The seal may be disposed between the housing and the suction outlet of the inlet mechanism. The seal may include upper and lower surfaces that are angled relative to one another to provide the decline angle with the receiver coupling portion oriented at a vertical angle. The upper and lower surfaces may be arranged at an angle within the range of two to seven degrees, and more particularly five degrees. The seal may include a friction ring.

Therefore, according to a first aspect of the present disclosure, the medical waste collection system for collecting medical waste material through the manifold during the medical procedure includes the waste container and the vacuum source configured to provide the vacuum on the waste container. The medical waste collection system also includes the receiver coupled to the waste container. The receiver includes the housing having an opening into which the manifold is configured to be inserted. The housing further includes the receiver outlet and an inlet mechanism coupled to the housing so as to be moveable in proximal and distal directions along an inlet axis. The inlet mechanism includes the suction inlet. The suction outlet is in fluid communication with the suction inlet. The sled assembly is moveably coupled to the housing and operably coupled to the inlet mechanism. The sled assembly is configured to be moved in the proximal direction during insertion of the manifold into the receiver in the proximal direction to facilitate the inlet mechanism moving correspondingly in the distal direction to establish fluid communication between the suction outlet and the receiver outlet. The lock assembly is coupled to the housing and configured to lock the manifold within the receiver in the fully inserted position. The actuator is coupled to the lock assembly and is axially moveable relative to the housing. The actuator is configured to receive an axial input from the user to cause the lock assembly to unlock the manifold.

In certain implementations, the lock assembly may include an arm rotatably coupled to the housing. The lock assembly may also include an arm biasing member biasing the arm to the locked configuration in which the arm abuts the manifold in the fully inserted position to prevent distal movement of the manifold and the sled assembly. The actuator may include the ramped surface configured to abut the arm and rotate the arm in opposition to the biasing member away from the manifold to permit movement of the manifold and the sled assembly in the distal direction. The sled assembly may include the sled body being configured to abut the manifold. The sled body may be movable to at least the proximal position when the manifold is in the fully inserted position. The sled body may also be movable to the distal position. The sled body may be moveable with the manifold while the manifold is disposed within the opening of the receiver. The sled assembly may include the sled biasing member coupled to the sled body. The sled biasing member may be configured to bias the sled body distally against the arm while the arm is in the locked configuration. The sled biasing member may be configured to move the sled body and the manifold distally from the proximal position and the fully inserted position, respectively, responsive to movement of the arm to an unlocked configuration. The sled body may include an arm retention surface configured to abut the arm of the lock assembly and retain the arm of the lock assembly in an unlocked configuration while the sled body is in the distal position.

According to the second aspect of the present disclosure, the medical waste collection system for collecting medical waste material through the manifold during the medical procedure includes the waste container and the vacuum source configured to provide the vacuum on the waste container. The waste collection system also includes the receiver coupled to the waste container. The receiver includes the housing having an opening into which the manifold is configured to be inserted. The housing includes the receiver outlet and an inlet mechanism coupled to the housing. The inlet mechanism includes the suction inlet. The suction outlet is in fluid communication with the suction inlet. The inlet mechanism is moveable between the first position where the suction outlet and the receiver outlet are not in fluid communication and the second position where the suction outlet and the receiver outlet are in fluid communication. The inlet lock assembly has the latch configured to be moveably coupled to the housing. The biasing member biases the latch to the locked position in which movement of the inlet mechanism to the second position is prevented. The latch is configured to be moveable responsive to abutting engagement with the manifold during insertion of the manifold in the receiver from the locked position to an unlocked position in which movement of the inlet mechanism to the second position is permitted.

In certain implementations, the latch may be pivotably coupled to the housing about the latch axis. The latch may include the head portion and the tail portion opposite the head portion from the latch axis. The tail portion may be longer than the head portion. The inlet mechanism may include an inlet base moveable between the first and second positions. The latch may be configured to abut the inlet base in the locked position to prevent the inlet base from moving to the second position. The inlet base may define the cavity for receiving the latch when the latch is in the unlocked position and the inlet base is in the second position. A sled assembly may be moveably coupled to the housing and operably coupled to the inlet mechanism. The sled assembly may be configured to be moved in the proximal direction during insertion of the manifold into the receiver in the proximal direction to facilitate the inlet mechanism moving correspondingly in the distal direction to the second position. The inlet lock assembly of the second aspect may be provided in combination with the actuator of the first aspect, and optionally, any of its corresponding implementations.

According to the third aspect of the present disclosure, the medical waste collection system for collecting medical waste material through the manifold during the medical procedure includes the waste container and the vacuum source configured to provide the vacuum on the waste container. The medical waste collection system also includes the receiver coupled to the waste container. The receiver includes the housing having an opening into which the manifold is configured to be inserted. The housing includes the receiver outlet. The inlet mechanism is coupled to the housing so as to be moveable in proximal and distal directions. The inlet mechanism includes the suction inlet. The suction outlet is in fluid communication with the suction inlet. The sled assembly is moveably coupled to the housing and operably coupled to the inlet mechanism. The sled assembly is configured to be moved in the proximal direction during insertion of the manifold into the receiver to facilitate the inlet mechanism moving correspondingly in the distal direction to establish fluid communication between the suction outlet and the receiver outlet. The motion conversion assembly includes the cam mechanism operatively coupling the sled assembly and the inlet mechanism to facilitate the respective corresponding movements of the sled assembly and the inlet mechanism in the proximal and distal directions.

In certain implementations, the cam mechanism may include the cam body rotatably coupled to the housing about the cam center axis. The cam body has an eccentric surface with the plurality of points of the eccentric surface being spaced from the cam center axis at different radial distances. The inlet mechanism may include an inlet base defining an inlet slot. The cam mechanism may include an inlet mechanism engagement pin extending from the cam body and being received in the inlet slot. The inlet mechanism engagement pin may be configured to move within the inlet slot and abut the inlet base to move the inlet mechanism proximally and distally in response to rotation of the cam body. The sled assembly may include the sled body defining the sled slot. The cam mechanism may include the sled engagement pin extending from the cam body and being received in the sled slot. The sled engagement pin may be configured to move within the sled slot and abut the sled body to move the sled assembly proximally and distally in response to rotation of the cam body. The motion conversion assembly may include the cam follower mechanism configured to provide resistance to rotation of the cam body. The cam follower mechanism may include the lever rotatably coupled to the housing about the lever axis spaced from the cam center axis. The cam follower mechanism may include the roller rotatably coupled to the lever and configured to be in direct contact with the eccentric surface of the cam body. The cam follower mechanism may include the biasing element coupled to the lever to bias the roller into contact with the eccentric surface of the cam body and to provide resistance to rotation of the cam body. The eccentric surface may include the first point at the first radial distance from the cam center axis, the second point at the second radial distance from the cam center axis, and the third point at the third radial distance from the cam center axis. The second radial distance may be greater than the first and third radial distances. The second point may be circumferentially disposed between the first and third points. The motion conversion assembly of the third aspect may be provided in combination with the actuator of the first aspect and/or the inlet lock assembly of the second aspect, and optionally, any of their corresponding implementations.

According to the fourth aspect of the present disclosure, the medical waste collection system for collecting medical waste material through the manifold during the medical procedure includes the waste container having the waste container inlet. The medical waste collection system also includes the vacuum source configured to provide the vacuum on the waste container. The medical waste collection system further includes the receiver coupled to the waste container. The receiver has the housing including an opening into which the manifold is configured to be inserted at the decline angle relative to horizontal. The housing further includes the receiver outlet. The inlet mechanism is coupled to the housing and includes the suction inlet and the suction outlet in fluid communication with the suction inlet. The inlet mechanism is moveable along an inlet axis at the decline angle between the first position in which the suction outlet and the receiver outlet are not in fluid communication and the second position in which the suction outlet and the receiver outlet are in fluid communication. The conduit is coupled to and extends between the receiver outlet and the waste container inlet to facilitate the transfer of waste material from the receiver outlet to the waste container. The conduit has the receiver coupling portion extending from the receiver outlet along the conduit axis that is oblique to the inlet axis.

In certain implementations, the seal may be coupled to the housing to cover the receiver outlet. The suction outlet may extend along the suction outlet axis that is oblique to the conduit axis. The suction outlet axis may be perpendicular to the inlet axis. The conduit of the fourth aspect may be provided in combination with the actuator of the first aspect, the inlet lock assembly of the second aspect, and/or the motion conversion mechanism of the third aspect, and optionally, any of their corresponding implementations.

In certain implementations, the sled assembly may be moveably coupled to the housing and operably coupled to the inlet mechanism. The sled assembly may be configured to be moved in the proximal direction during insertion of the manifold into the receiver in the proximal direction to facilitate the inlet mechanism moving correspondingly in the distal direction to the second position. The sled assembly may be configured to be moved in the distal direction opposite the proximal direction during removal of the manifold from the receiver to facilitate the inlet mechanism moving correspondingly in the proximal direction to break fluid communication between the suction outlet and the receiver outlet. A claw may be coupled to the sled assembly. The claw may be configured to selectively engage the manifold and facilitates movement of the sled assembly in the distal direction during removal of the manifold from the receiver. An electronics module may be in communication with the vacuum source. The receiver may include the sensor in communication with the electronics module. The sensor may be configured to output the signal indicative of the position of the sled assembly in the proximal and distal directions. The electronics module may be configured to control the vacuum source based on the signal from the sensor. A magnet may be disposed on the sled assembly and configured to be detected by the sensor. The electronics module may be configured to prevent operation of the vacuum source based on the signal from the sensor when the manifold is not inserted into the receiver to the fully inserted position. A first barrier may be pivotably coupled to the housing. The first biasing element may be coupled to the first barrier and configured to bias the first barrier towards the closed position to selectively cover at least the portion of the opening of the receiver. A second barrier may be pivotably coupled to the sled assembly and positioned proximal to the first barrier. The second biasing element may be coupled to the second barrier and configured to bias the second barrier towards the closed position. Movement of the inlet mechanism in the distal direction may facilitate moving the second barrier from the closed position to an open position in which the suction inlet of the inlet mechanism is exposed to the manifold being inserted.

### BRIEF DESCRIPTION OF THE DRAWINGS

Advantages of the present disclosure will be readily appreciated as the same becomes better understood by reference to the following detailed description when considered in connection with the accompanying drawings.
Figure 1 is a perspective view of a medical waste collection system.
Figure 2 is a perspective view of a receiver coupled to a waste container of the medical waste collection system.
Figure 3 is a perspective view of the receiver and a manifold.
Figure 4 is a partially exploded view of the receiver in which certain internal components of the receiver and the manifold are illustrated.
Figure 5 is a sectional elevation view of the receiver taken along lines 5-5 of Figure 3 without the manifold.
Figure 6 is perspective view of a portion of the receiver with a top removed.
Figure 7 is a sectional elevation view of the receiver with the manifold preparing to enter the receiver.
Figure 8 is a sectional elevation view of a portion of the receiver with a motion conversion assembly shown in a first position and a sled assembly in a first configuration.
Figure 9 is a sectional elevation view of a portion of the receiver with the motion conversion assembly shown in a second position and the sled assembly in a second configuration.
Figure 10 is a sectional elevation view of a portion of the receiver with the motion conversion assembly shown in a third position and the sled assembly in a third configuration.
Figure 11 is a sectional elevation view of a portion of the receiver with the motion conversion assembly shown in a third position and the sled assembly in a fourth configuration.
Figure 12 is a perspective view of a portion of the receiver and a manifold.
Figure 13 is a sectional view of the receiver taken along the lies 13-13 of Figure 3. A portion of a manifold is shown as being partially inserted into the receiver.
Figure 14 is a perspective view of a portion of the receiver with the sled assembly in the first configuration and the manifold initiating contact with the sled assembly.
Figure 15 is a sectional plan view of the manifold and receiver with the manifold fully inserted in the receiver.
Figure 16 is a detailed sectional plan view of Figure 15 illustrating a locking arm of the receiver and the manifold in a fully inserted position.
Figure 17 is a detailed sectional plan view of the locking arm engaging a lateral surface of the manifold and the manifold being in a partially inserted position.
Figure 18 is a detailed sectional plan view of the locking arm engaging the sled assembly and the sled assembly being in the first configuration.
Figure 19 is a perspective view of the motion conversion assembly and an inlet mechanism.

### DETAILED DESCRIPTION

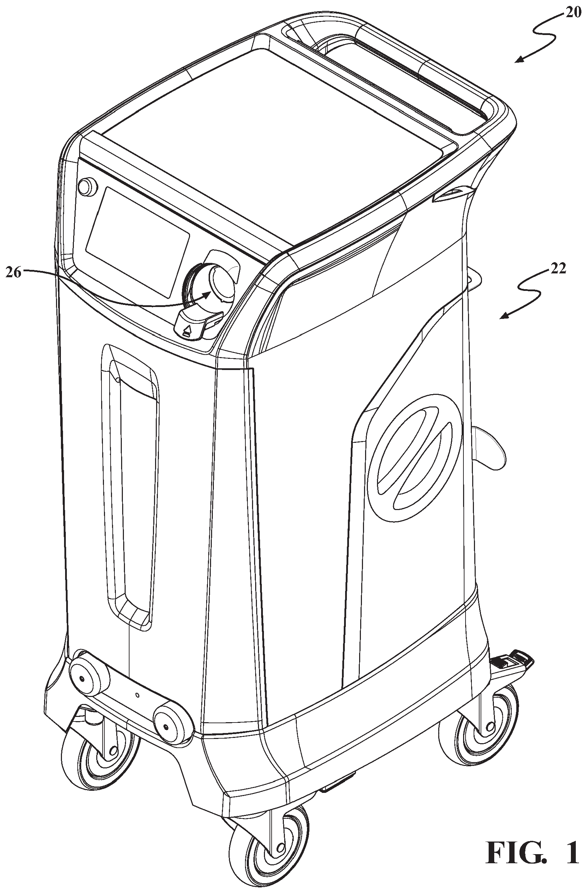

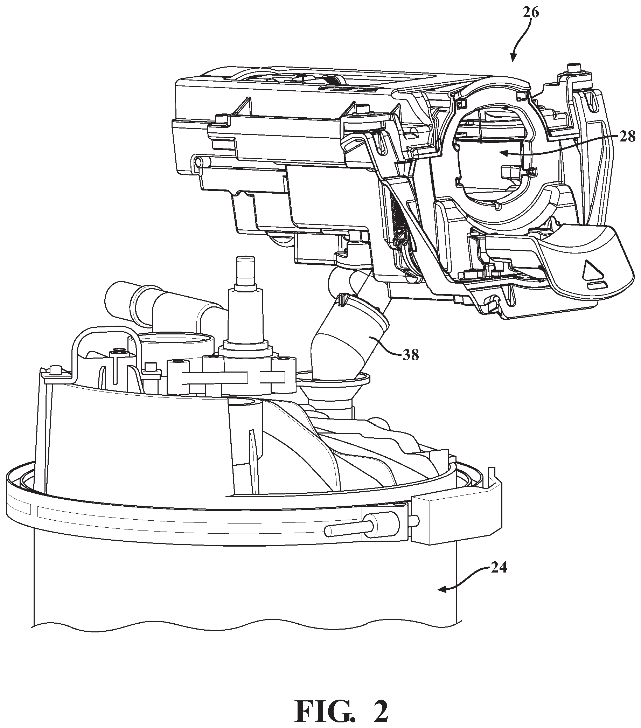

Figure 1 shows a medical waste collection system 20 for collecting the waste material generated during medical procedures, and more particularly surgical procedures. The medical waste collection system 20 collects the waste material and/or stores the waste material until it is necessary or desired to off-load and dispose of the waste material. The medical waste collection system 20 may comprise a cart 22 including wheels for moving the cart along a floor surface within a medical facility. With further reference to Figure 2, the medical waste collection system 20 includes at least one waste container 24 defining a waste volume for collecting and storing the waste material, and a receiver 26 coupled to the waste container 24 with a conduit 38. A vacuum pump is supported on the cart and configured to draw suction on the waste container(s) 24 and the receiver 26. Suitable construction and operation of several subsystems of the medical waste collection system 20 are disclosed in commonly-owned International Patent Publication No. WO 2020/027850, United States Patent Publication No. 2005/0171495, International Patent Publication No. WO 2007/070570, International Patent Publication No. WO 2014/066337, and International Patent Publication No. WO 2017/112684, the entire contents of each are hereby incorporated by reference.

The receiver 26 includes an inlet mechanism 32 that is movable to be coupled with a manifold 30 to establish fluid communication between the manifold 30 and the waste container 24 in a manner to be further described. The inlet mechanism 32 may include a suction inlet 33, and a suction outlet 34 in fluid communication with the suction inlet 33. The suction inlet 33 is configured to be arranged in fluid communication with the manifold 30, and the suction outlet 34 is configured to be arranged in fluid communication with a receiver outlet 36.

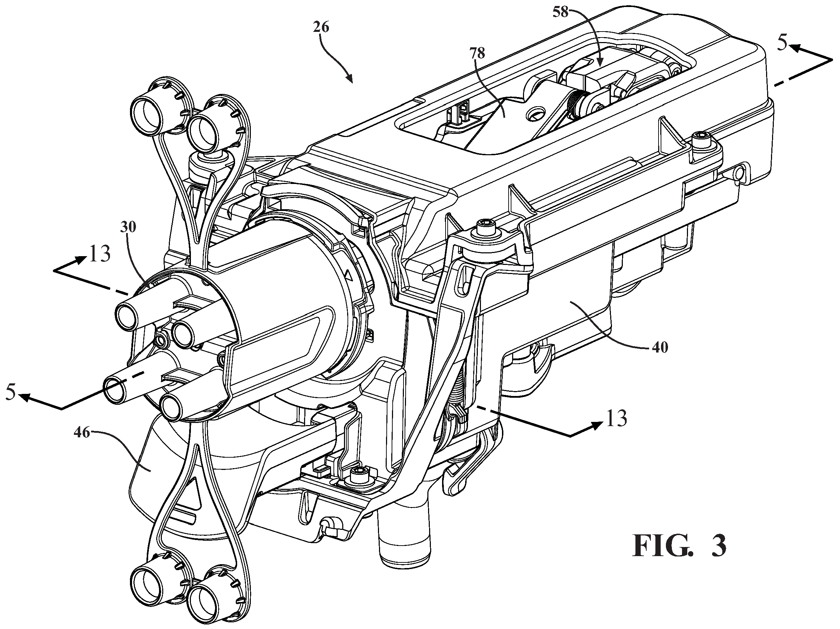

Referring now to Figures 3-7, the receiver 26 includes a housing 40. The housing 40 may define an opening 28 sized to removably receive a manifold 30. Certain portions of the manifold 30 may be illustrated and described herein, and otherwise may be similar to or the same as those disclosed in commonly-owned United States Patent No. 10,471,188, issued November 12, 2019, the entire contents of which are hereby incorporated by reference. The receiver 26 may include a first barrier 44 positioned to cover the opening 28. The first barrier 44 may be biased to a closed position, as shown, with the bias metered to be overcome by anticipated forces associated with insertion of the manifold 30 through the opening 28. An actuator 46 is movably coupled to the housing 40, and further coupled to a lock assembly 48 configured to permit removal of the manifold 30 from the receiver 26. As such, the actuator 46 may appear akin to an "eject button" configured to be pushed in a proximal direction. In other configurations, the actuator 46 may be configured to receive a pull input to be moved in a distal direction.

The receiver 26 may include subcomponents and subassemblies configured to engage complementary features of the manifold 30 during insertion and removal of the manifold 30. These may include the lock assembly 48 and the inlet mechanism 32, and further include a sled assembly 58, an inlet lock assembly 60, claws 62, and a motion conversion assembly 64. The inlet mechanism 32 may be configured to move in the proximal-to-distal direction in an opposite direction of that of the sled assembly 58 during insertion and removal of the manifold 30 into and from the receiver 26, respectively. The inlet lock assembly 60 may be configured to prevent distal movement of the inlet mechanism 32 during an attempted insertion of a manifold lacking the requisite features. The claws 62 are movably coupled to the housing 40 and configured to articulate inwardly to engage the manifold 30. The motion conversion assembly 64 is configured to convert movement of the sled assembly 58 into movement of the inlet mechanism 32. The motion conversion assembly 64 may be configured to provide tuned resistance during insertion of the manifold 30 into the receiver, and further provide initial return movement of the manifold 30 after disengaging the lock assembly 48 from the manifold 30 via the actuator 46.

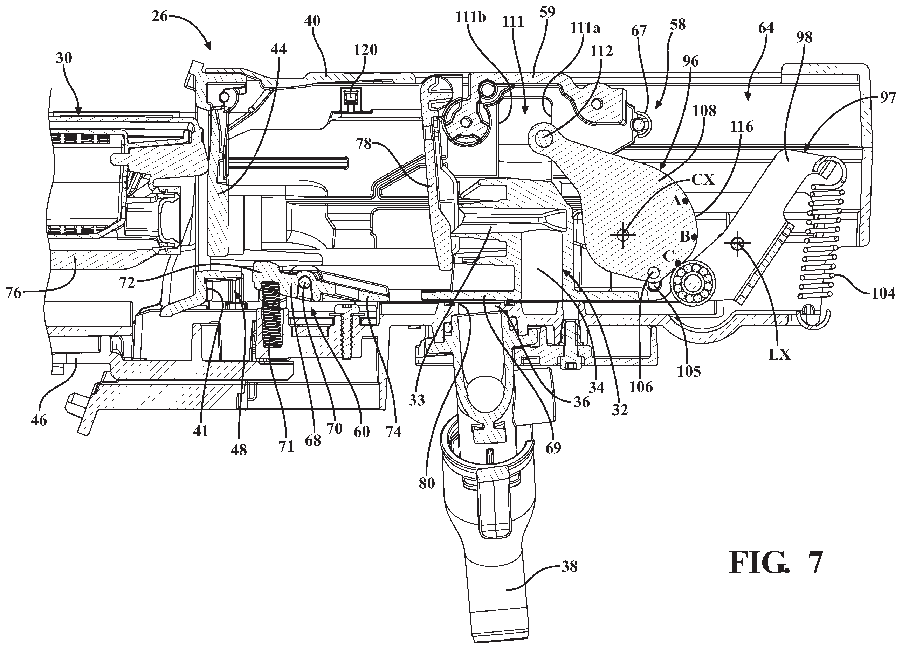

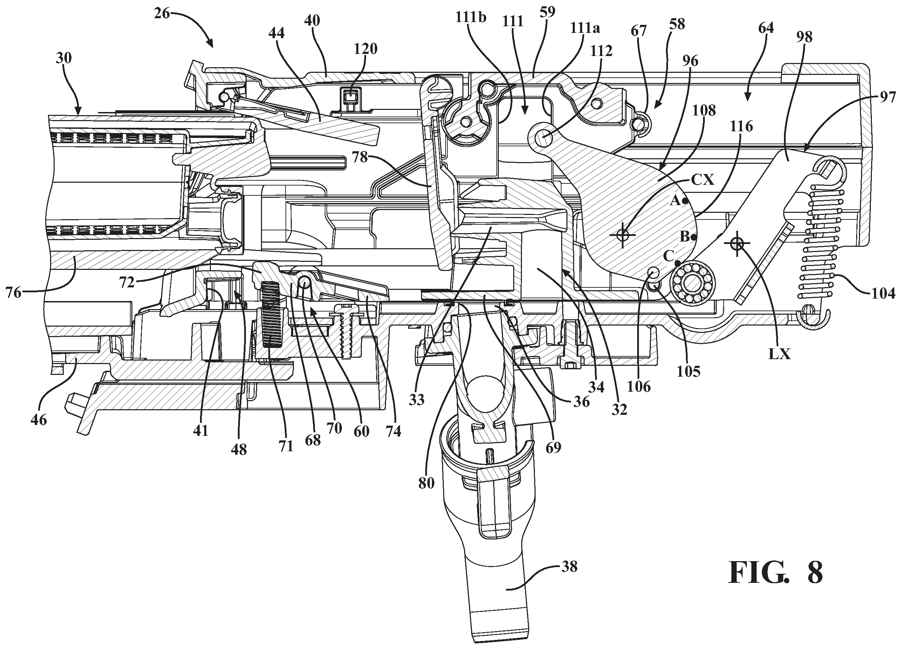

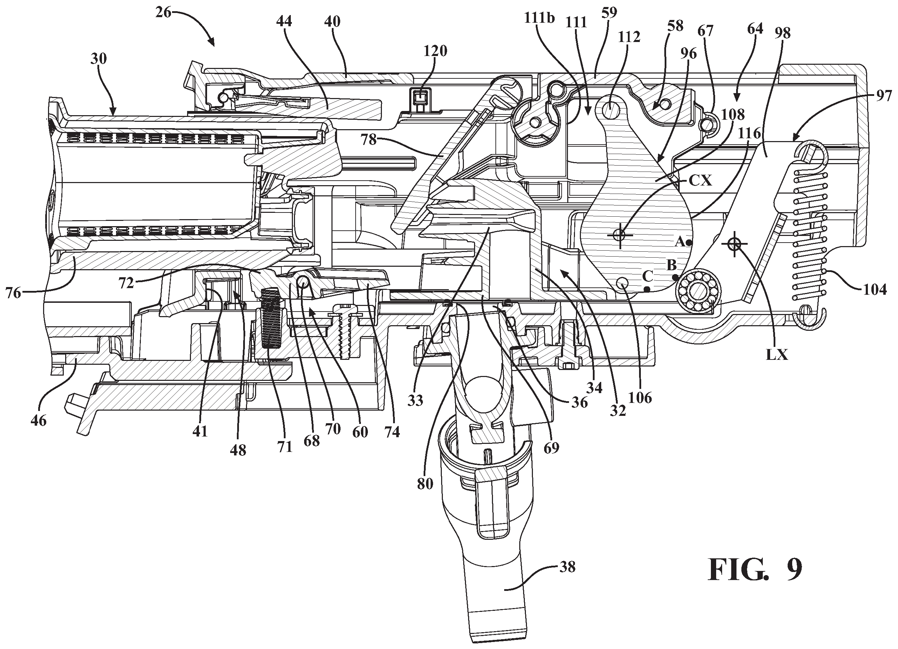

With reference to Figures 7-11, the subcomponents and subassemblies are further described with reference to several positions associated with insertion of the manifold 30 into the receiver 26, and further with reference to the related disclosure of the aforementioned United States Patent No. 10,471,188. The manifold 30 is oriented for insertion into the opening 28 of the receiver 26 and directed through the opening 28 so as to move the first barrier 44 to an open position (Figure 8). The manifold 30 is further advanced to a position in which a spine 76 of the manifold 30 engages the inlet lock assembly 60 (Figure 9). The inlet lock assembly 60 may include a latch 68 pivotably coupled to a lower wall of the housing 40 of the receiver 26. The inlet lock assembly 60 may be biased to a locked position by a latch biasing member 71 in which potential interference between a base 69 of the inlet mechanism 32 and the latch 68 of the inlet lock assembly 60 would prevent distal movement of the inlet mechanism 32 necessary to establish fluid communication between the inlet mechanism 32 and both the manifold 30 and the receiver outlet 36.

The latch 68 is pivotable about a latch axis 70 and includes a head portion 72, and a tail portion 74 positioned opposite the latch axis. The tail portion 74 may be longer than the head portion 72. The latch biasing member 71 may be a coil spring disposed between the head portion 72 and the lower wall of the housing 40. The spine 76 of the manifold 30 engaging the latch 68 moves the inlet lock assembly 60 from a locked position in which the base 69 of the inlet mechanism 32 would collide with the tail portion 74, to an unlocked position in which further distal movement of the inlet mechanism 32 is permitted. More particularly, a ramped surface of the spine 76 may directly contact the head portion 72 at a deflection angle so as to cause pivoting of the latch 68 about the latch axis 70. The pivoting of the latch 68 moves the tail portion 74 out of potential interference with the base 69 that is correspondingly approaching from the proximal direction. The base 69 of the inlet mechanism 32 may define a cavity for receiving the tail portion 74 of the latch 68 when the latch 68 is in the unlocked position. With the inlet lock assembly 60 in the unlocked configuration, the manifold 30 may be moved to the fully inserted position in which the base 69 is situated within the cavity and fluid communication is established between the suction outlet 34 and the receiver outlet 36. In other words, should the manifold 30 lack the spine 76 and its characteristics relative to other features of the manifold 30 to be described, interference between the inlet mechanism 32 and the inlet lock assembly 60 may cause "binding" of the receiver 26 and prevent the fluid communication between the suction outlet 34 and the receiver outlet 36. This may be intentional such that only genuine manifolds may be used with the receiver 26.

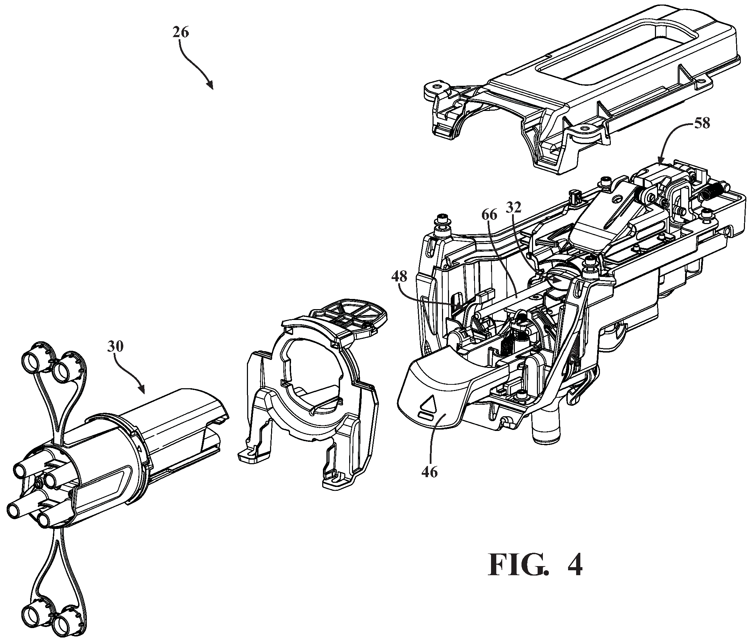

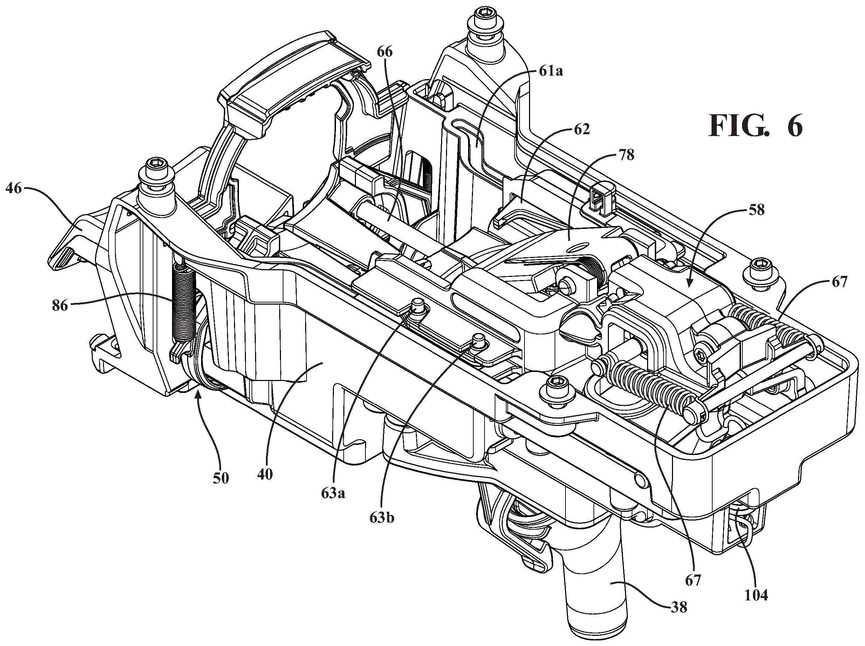

The sled assembly 58 of the receiver 26 may include a sled body 59. The sled body 59 may be slidably disposed on rails 66 extending within the housing 40 of the receiver 26 in the proximal-to-distal direction, as best shown in Figures 4 and 6. The manifold 30 is further advanced by the user into the opening 28 of the receiver 26 until one or more arms 90 of the manifold 30 engage the sled body 59. More specifically, the sled body 59 may define one or more slots 91 for receiving the one or more arms 90 of the manifold 30. The sled assembly 58 is moved proximally along the rails 66 by the manifold 30 engaging the sled body 59. In some configurations, the manifold 30 engages the sled assembly 58 before the spine 76 of the manifold 30 engages the latch 68 as previously described. In other configurations, the manifold 30 engages the sled assembly 58 after the spine 76 of the manifold 30 engages the latch 68. In still other configurations, engagement of the manifold 30 to the sled assembly 58 and engagement of the spine 76 of the manifold 30 with the latch 68 occur simultaneously.

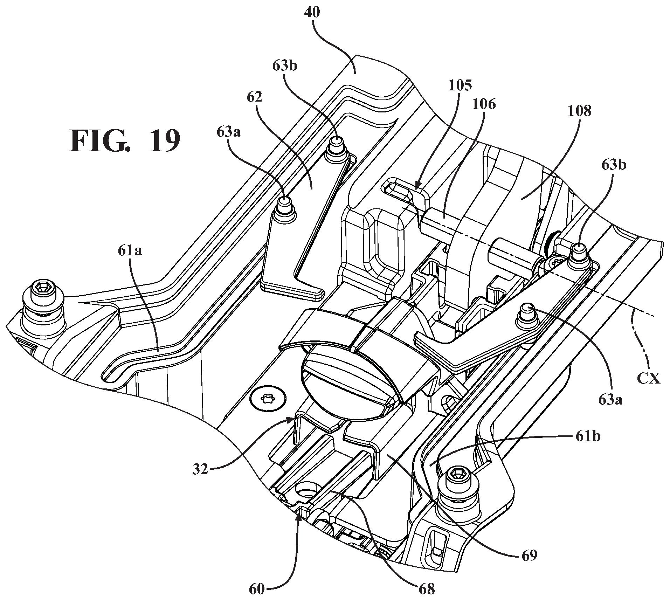

The claws 62 may engage catches (not identified) of the manifold 30 with continued insertion of the manifold 30 within the opening 28 of the receiver 26. The claws 62 may be coupled to the sled assembly 58, and the movement of the sled assembly 58 may result in the claws 62 articulating inwardly to engage the catches of manifold 30. More specifically, Figure 19 shows the housing 40 may defining one or more channels 61a, 61b extending generally in proximal-to-distal directions on either side of the sled assembly 58. Each of the claws 62 may comprise first and second claw pins 63a, 63b that are slidable within each channel 61a, 61b. The channels 61a, 61b guide the path of the pins 63a, 63b as the sled assembly 58 moves proximally and distally. The first and second claw pins 63a, 63b and channels 61a, 61b cooperate to cause the claws 62 to articulate inwardly toward the manifold 30 responsive to movement of the sled assembly 58 in the proximal direction. The engagement between the claws 62 and the catches transfer forces from the manifold 30 to the sled assembly 58, particularly during removal of the manifold 30 from the receiver 26 by the user.

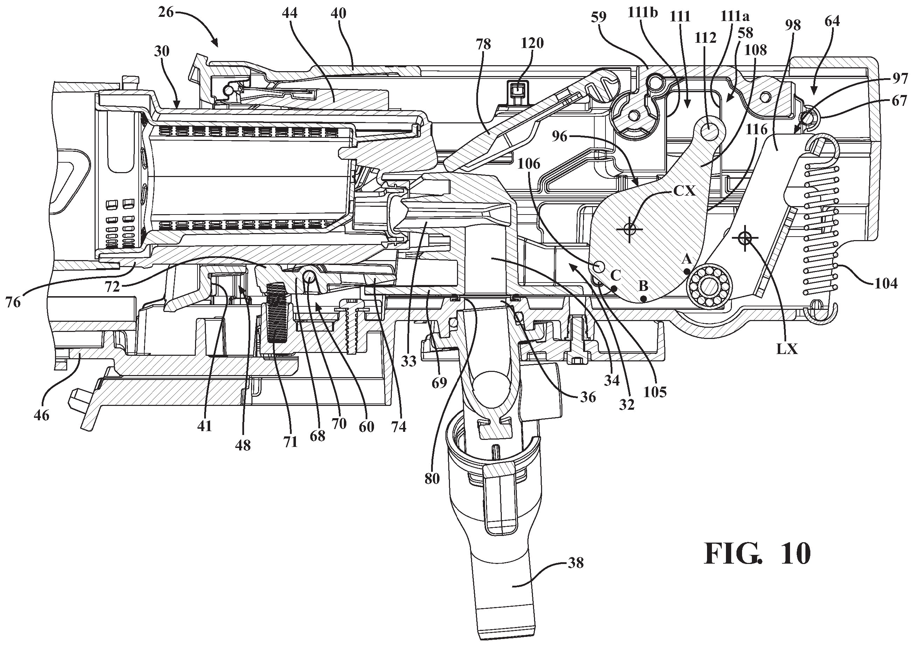

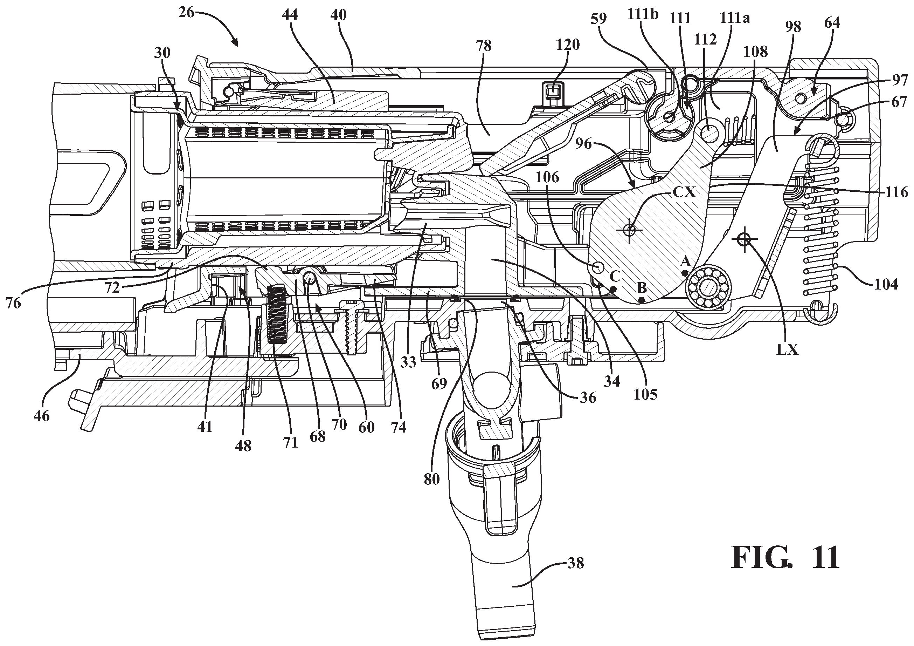

With the further movement of the sled assembly 58 in the proximal direction, and thus, movement of the inlet mechanism 32 in the distal direction, the suction outlet 34 of the inlet mechanism 32 moves towards alignment with the receiver outlet 36. A second barrier 78 is at least partially moved from a closed position (see Figures 7 and 8) to an open position as shown in Figures 10 and 11 by the inlet mechanism 32 moving in the distal direction. The second barrier 78 in the closed position prevents the user from see or touching the inlet mechanism 32 even if the first barrier 44 is manually manipulated to the open position. The manifold 30 assumes the fully inserted position within the receiver 26, as shown in Figure 11. In this position, the inlet mechanism 32 engages the manifold 30, for example, by extending through a seal covering an outlet opening of the manifold 30. Further, the suction outlet 34 is aligned with the receiver outlet 36 to establish fluid communication between the manifold 30 and the receiver outlet 36, and thus, the waste container 24.

To selectively secure the manifold 30 in the receiver 26, the lock assembly 48 is provided. The lock assembly 48 may include an arm 50 rotatably coupled to the housing 40 and an arm biasing member 86, such as a spring. The arm biasing member 86 biases the arm to a locked configuration where the arm 50 is configured to abut the manifold 30 in the fully inserted position to prevent distal movement of the manifold 30 and a sled assembly 58. In other words, in the fully inserted position, the lock assembly 48 is moved from the unlocked configuration to the locked configuration to engage lock elements 82 of the manifold 30 and retain the manifold 30 in the proximal-to-distal direction, and in particular against distal forces from the motion conversion assembly 64 to be described.

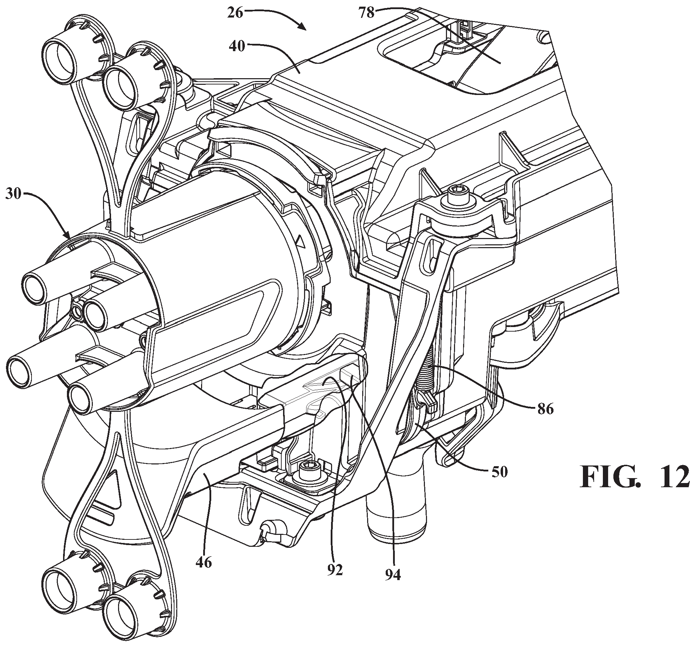

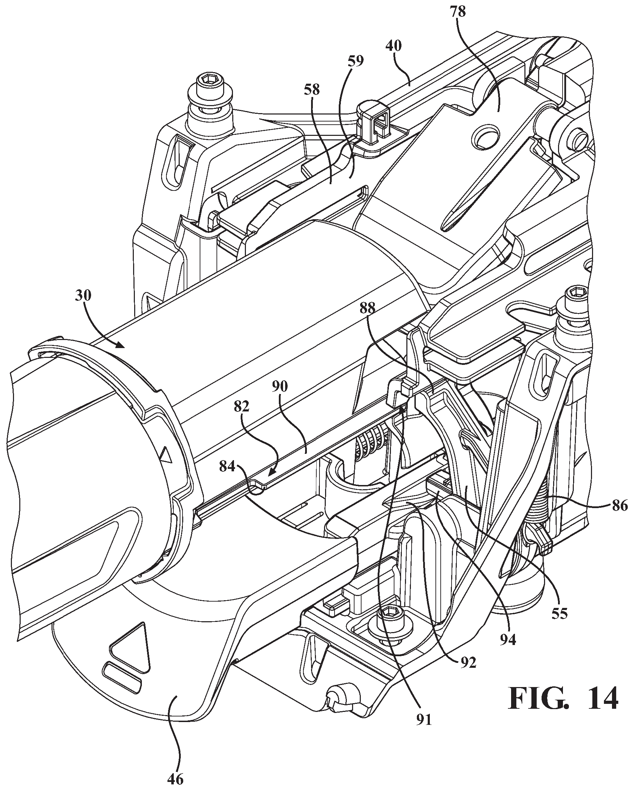

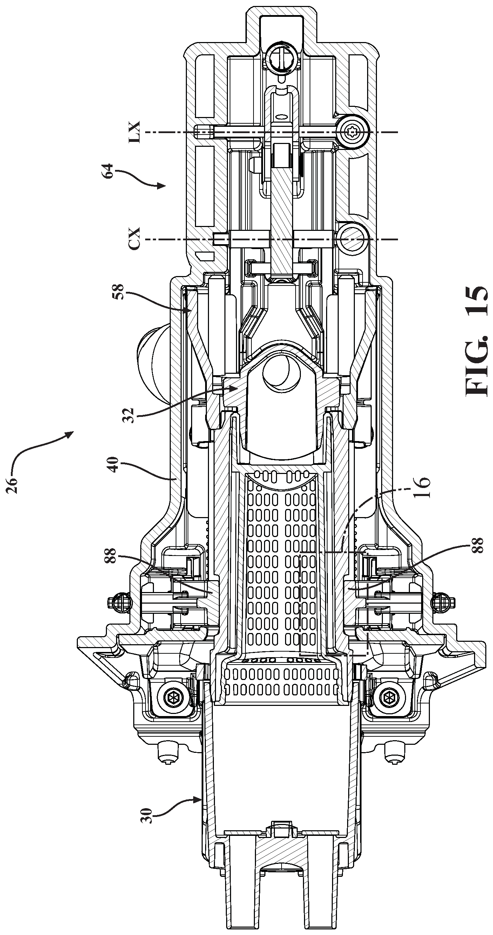

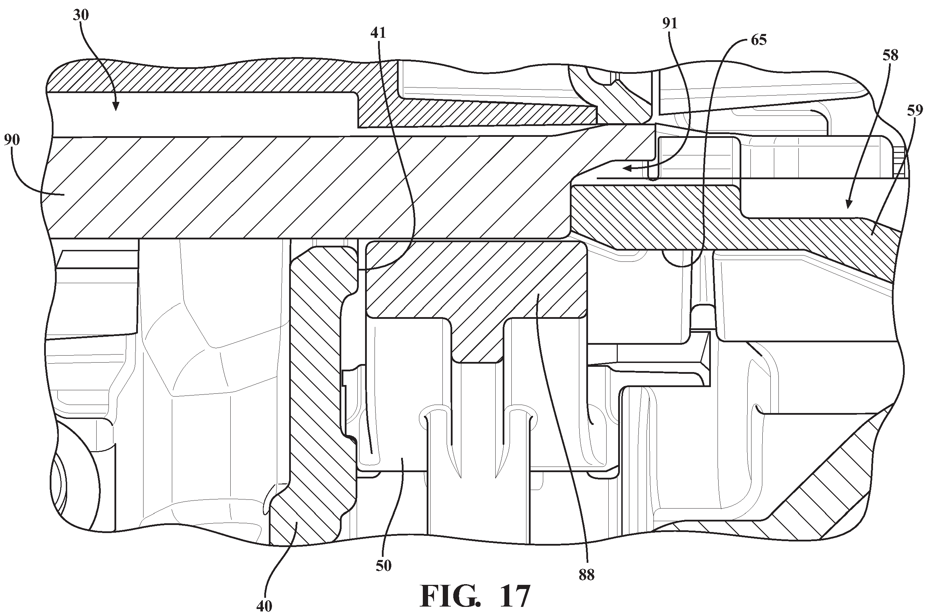

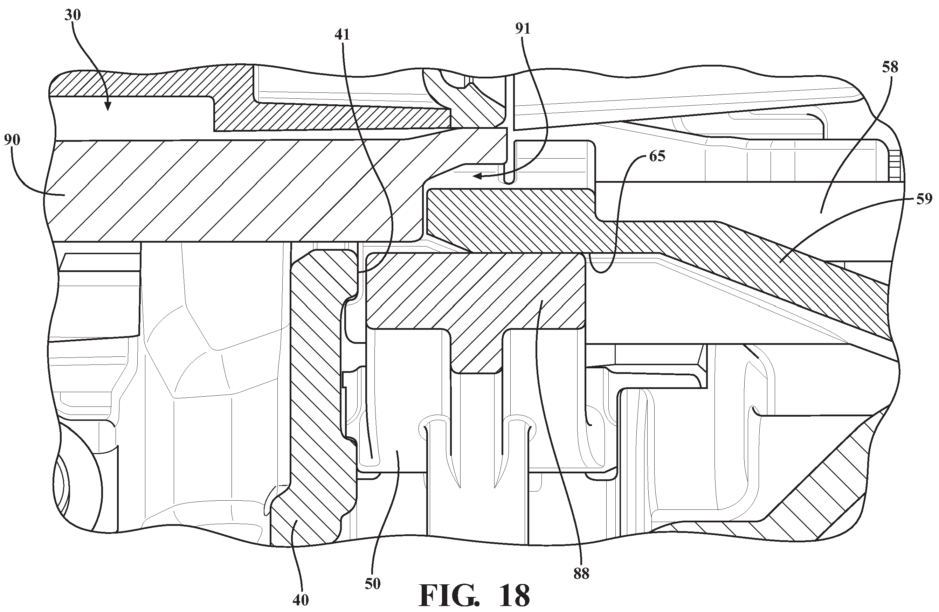

Referring now to Figures 12-18, the arms 50 of the lock assembly 48 are pivotably coupled to the housing 40 and positioned opposite a void space into which the manifold 30 is to be situated. The arms 50 are biased to the locked position with springs 86 coupling the arms 50 and the housing 40. For example, the arms 50 may each include a shoulder 88 that is biased inwardly. The sled body 59 has an arm retention surface 65 configured to abut the shoulder 88 of the arm 50 of the lock assembly 48 and retain the arm 50 in an unlocked configuration while the sled body 59 is in the distal position. In other words, the arm retention surface 65 is configured to hold the arms 50 in an unlocked configuration (e.g., articulated outwardly) until the manifold 30 engages the sled assembly 58 to move the sled assembly 58 proximally away from the arms 50. Further advancement of the manifold 30 into the receiver 26 includes the shoulders 88 of the arms 50 being biased by the springs 86 to slide off of the arm retention surfaces 65 and "ride" on lateral surfaces of the arms 90 of the manifold 30, as best shown in Figure 17. The shoulders 88 of the arms 50 continue to "ride" on lateral surfaces of the arms 90 of the manifold 30 until the manifold 30 is fully inserted. When the manifold 30 is fully inserted, the arms 50 encounter the lock elements 82 of the manifold 30, which may be considered a proximal end of the arms that outwardly project from a housing of the manifold 30. The continued bias in the springs 86 moves the arms 50 to pivot the shoulders 88 inwardly into engagement with the lock elements 82 of the manifold 30, as shown in Figure 7. Further, the springs 86 may be design with a spring constant or other suitable characteristic to pivot the shoulders 88 inwardly with sufficient speed for the shoulders 88 to impact the housing of the manifold 30. The impact is of sufficient force to provide audible and/or tactile feedback to the user inserting the manifold 30 that the manifold 30 is fully inserted and locked in position. The audible feedback may be a "clicking" sound from the impact, and the tactile feedback may be secondary to the shoulders 88 impacting the manifold 30 that may be plastic in construction and being held by the user's hand.

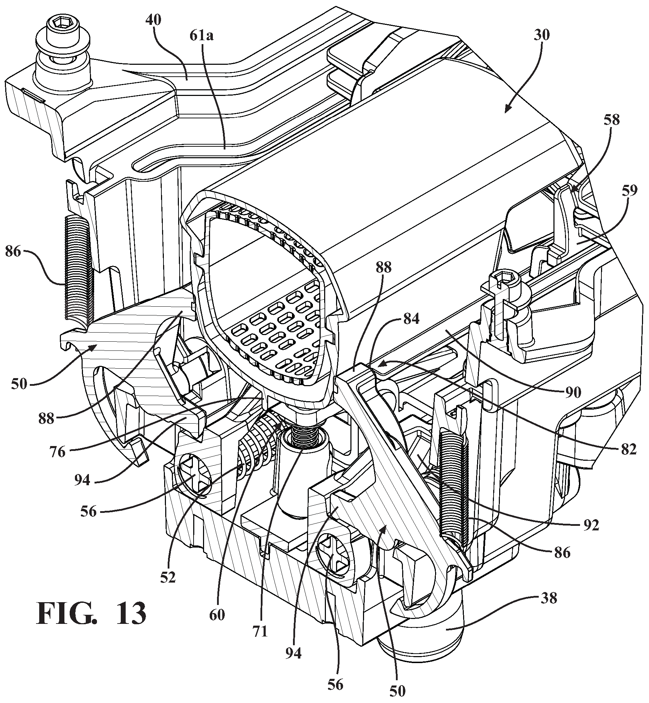

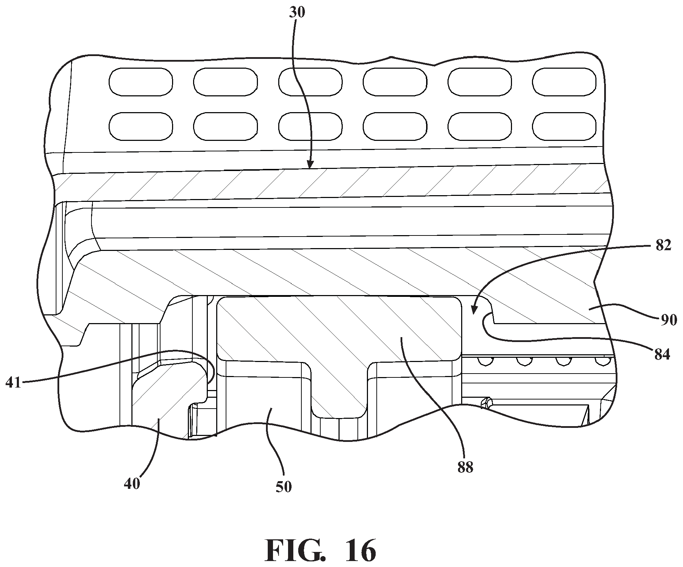

With the manifold 30 fully inserted, the manifold 30 may not be removed with the lock assembly 48 in the locked configuration. Figures 13 and 16 show the lock elements 82 including distally-directed surfaces 84 positioned adjacent to the shoulders 88. Interference between the distally-directed surfaces 84 and the shoulders 88 prevent distal movement of the manifold 30 within the receiver 26, particularly against distal forces from a sled biasing member 67 coupled to the motion conversion assembly 64 to be described.

The shoulders 88 of the arms 50 may be configured to engage the housing 40 in the locked configuration. More particularly, Figure 16 shows the shoulders 88 compressed or "sandwiched" between the distally-directed surfaces 84 of the manifold 30 and an inner surface 41 of the housing 40. Forces in the distal direction from the manifold 30 (from the bias from the motion conversion assembly 64) are counteracted by the housing 40, thereby advantageously making the lock assembly 48 more robust in operation. The arms 50 are configured to pivot inwardly and outwardly as described, and thus the forces in the distal direction are orthogonal to the pivot. The arms 50 of the lock assembly 48 may further provide for slight pivoting in the distal direction to permit the shoulders 88 to directly contact the inner surface 41 of the housing 40 with the manifold 30 inserted in the receiver 26 and the lock assembly 48 in the locked configuration. Absent the advantageous arrangement in which the housing 40 counteracts the forces, the pivots of the arms 50 themselves may otherwise need to be designed to withstand the appreciable forces in the distal direction, which may require larger or heavier components in an generally spaced-constrained stack up. Furthermore, the arms 50 being relatively lighter and more compact in form results in improved performance of the springs 86 to rapidly pivot the shoulders 88 inwardly with sufficient speed for the shoulders 88 to impact the housing of the manifold 30.

Once it is desired to remove the manifold 30 from the receiver 26, for example, subsequent to use of the medical waste collection system 20 during a surgical procedure, the actuator 46 is actuated (e.g., pulled or pushed). Referring to Figures 12-14, the actuator 46 may include a ramped surface 92 configured to abut the arm 50 and rotate the arm 50 in opposition to the arm biasing member 86 away from the manifold 30 to permit movement of the manifold 30 and the sled assembly 58 in the distal direction. The actuator 46 itself may be biased to an outward distal position with a biasing element 52 so as to be operated by a push input. The biasing element 52 may be positioned between the actuator 46 and the housing 40. The actuator 46 and the biasing element 52 may be slidably disposed on rails 56 extending within the housing 40 of the receiver in the proximal-to-distal direction. In configurations where the actuator 46 is configured to receive a pull input, the actuator 46 may be biased to an inward proximal position with a biasing element positioned between the actuator 46 and the housing 40.

With continued reference to Figures 12 and 13, the proximal movement of the actuator 46 moves ramped surfaces 92 into engagement with fingers 94 of the arms 50. The fingers 94 and the shoulders 88 of the arms 50 are positioned opposite the pivot. The ramped surfaces 92 urge the fingers 94 generally upwardly and cause the shoulders 88 to pivot outwardly against the bias from the springs 86, as generally shown in Figure 6. The extent of outward pivoting of the shoulders 88 is at least sufficient to disengage the shoulders 88 from the distally-directed surfaces 84 of the lock elements 82 of the manifold 30. The disengagement permits the sled biasing member 67 to move sled assembly 58 and the manifold 30 an initial distance in the distal direction. The manifold 30 may be removed from the receiver 26 in reverse of the description of insertion above. Subsequent to the manifold 30 being removed, the latch 68 of the inlet lock assembly 60 returns to the locked position via the latch biasing member 71. Alternatively, the return may be based on the relative weight of the head portion 72 and tail portion 74.

The motion conversion assembly 64 is described with reference to Figures 7-11 and 19. The motion conversion assembly 64 may include a cam mechanism 96 and a cam follower mechanism 97. The cam mechanism 96 may include a cam body 108 (sometimes referred to merely as a "cam") rotatably coupled to the housing 40 about a cam center axis CX. The cam follower mechanism 97 may include a lever 98 rotatably coupled to the housing 40 about a lever axis LX spaced from the cam center axis CX. The cam follower mechanism also includes a roller 100 rotatably coupled to the lever and configured to be in direct rolling contact with the cam body 108. As mentioned above, the motion conversion assembly 64 is configured to convert movement of the sled assembly 58 into movement of the inlet mechanism 32. More particularly, the motion conversion assembly 64 is configured to convert movement of the sled assembly 58 in the proximal direction into movement of the inlet mechanism 32 in the distal direction during insertion of the manifold 30, and conversely convert movement of the sled assembly 58 in the distal direction into movement of the inlet mechanism 32 in the proximal direction during removal of the manifold 30.

To facilitate the conversion of motion between the cam body 108 and the inlet mechanism 32, the inlet base 69 may define an inlet slot 105. The cam mechanism 96 may include an inlet mechanism engagement pin 106 extending from the cam body 108 and radially spaced from the cam center axis CX. The inlet mechanism engagement pin 106 may be received in the inlet slot 105 and configured to move within the inlet slot 105 and abut the inlet base 69 to move the inlet mechanism 32 proximally and distally in response to rotation of the cam body 108. More specifically, the inlet slot 105 may be arranged vertically such that only the proximal-to-distal motion of the cam body 108 moves the inlet base 69. This arrangement of a pin in a slot permits the transfer of rotational motion to linear motion.

To facilitate the conversion of motion between the cam body 108 and the sled assembly 58, the sled body 59 may define a sled slot 111. The cam mechanism 96 may include a sled engagement pin 112 extending from the cam body 108 and radially spaced from the cam center axis CX. The sled engagement pin 112 may be received in the sled slot 111. The sled engagement pin 112 is configured to move within the sled slot 111 and abut the sled body 59 to move the sled assembly 58 proximally and distally in response to rotation of the cam body 108. More specifically, the sled slot 111 may be arranged vertically such that only the proximal-to-distal motion of the cam body 108 moves the sled body 59. This arrangement of a pin in a slot permits the transfer of rotational motion to linear motion. The arrangement of the sled engagement pin 112 and the inlet mechanism engagement pin 106 on opposite sides of the cam center axis CX promotes the opposite direction of movement between the sled assembly 58 and the inlet mechanism 32.

The sled body 59 may also have a proximal wall 111a and a distal wall 111b defining proximal and distal ends of the sled slot 111, respectively. The proximal and distal walls 111a, 111b allow the sled body 59 to continue to move proximally after the suction outlet 34 of the inlet mechanism 32 is aligned with the receiver outlet 36. As mentioned above, the sled biasing member 67 is coupled to the sled body 59 and the motion conversion assembly 64 and configured to bias the sled body 59 distally against the arm 50 while the arm 50 is in the locked configuration and while the suction outlet 34 is in fluid communication with the receiver outlet 36. More specifically, the sled biasing member 67 is coupled to the sled engagement pin 112 of the motion conversion assembly 64. With further reference to Figures 10 and 11, continued insertion of the manifold 30 and movement of the sled body 59 proximally, after the suction outlet 34 of the inlet mechanism 32 is aligned with the receiver outlet 36, causes the sled engagement pin 112 to move from the proximal wall 111a of the slot 111 toward the distal wall 111b of the slot 111 in opposition to the sled biasing member 67. This continues until the manifold 30 is in fluid communication with the suction inlet 33 and the arms 50 of the lock assembly 48 lock the manifold 30 in place. In other words, the sled body 59 continues moving proximally from its position in Figure 10 to its proximal-most position in Figure 11 to bring the manifold 30 into fluid communication with the suction inlet 33 without further distally moving the inlet mechanism 32. The sled biasing member 67 then urges the sled body 59 against the arm 50 while the arm 50 is in the locked configuration.

Moreover, the distance between the slot engagement pin 112 and the cam center axis CX may be greater than the distance between the inlet mechanism engagement pin 106 and the cam center axis CX. In one example, the distance between the slot engagement pin 112 and the cam center axis CX may be at least three times greater than the distance between the inlet mechanism engagement pin 106 and the cam center axis CX. The relative distances may advantageously provide a mechanical advantage to the user during insertion of the manifold 30 into the receiver 26 in which less insertion forces are required to move the sled assembly 58 and the inlet mechanism 32 as previously described.

The relative distances may be further tuned to provide a desired resistance profile during insertion of the manifold 30 into the receiver 26. In other words, encountering little or no resistance during insertion of the manifold 30 may leave the user with uncertainty as to whether it is fully or properly installed for use. Providing tactile feedback of smoothness and sturdiness of the receiver 26 is of importance, and the motion conversion assembly 64 of the present disclosure advantageously provides these characteristics with realization of the benefits of the aforementioned mechanical advantage. The roller 100 is rotatably coupled to a first end of the lever 98, and a second end of the lever 98 is resiliently coupled to the housing 40 with a lever biasing member 104. The lever axis LX may be spaced closer to the first or the second end of the lever 98 to impart the desired resistance during movement of the motion conversion assembly 64 between the first and second positions. The roller 100 is in direct contact with the cam 108, and the lever biasing member 104 pivots the lever 98 about the third pin 114 to maintain the direct contact with the cam body 108 for all positions of the cam body.

With continued reference to Figures 7-11 and 19, the cam 108 includes an eccentric surface 116 relative to the cam center axis CX. Certain points along the eccentric surface 116 are at greater distances from the cam center than others so as to tune the resistance profile as desired throughout the insertion of the manifold 30 into the receiver 26. Likewise, certain segments of the eccentric surface 116 may be flatter or have more curves to further tune the resistance profile. More particularly, the roller 100 engages the eccentric surface 116, and the relative distances of the certain points circumferentially arranged on the eccentric surface 116 results in greater pivoting of the lever 98 against the bias from the lever biasing member 104. For example, in the first position (see Figures 7 and 8), the roller 100 may directly contact the eccentric surface at Point C. As the cam body 108 is moved by the sled assembly 58 and rotates about the cam center axis (clockwise in Figures 7 and 8), and the point of contact between the roller 100 and the eccentric surface 116 moves from Point C towards Point B. The distance from Point B to the cam center axis CX is greater than the distance from Point C to the cam center axis CX, and further may be a maximum distance of the eccentric surface 116 from the cam center axis CX. As a result, the roller 100 is urged away from the cam center axis CX, and the lever 98 pivots correspondingly about the lever axis LX. Owing to the lever biasing member 104, the lever 98 provides counterpart forces on the cam mechanism 96 and through the component stack up - the counterpart forces are felt as resistance by the user. The resistance may be desirable for the early stages to require relatively more forceful insertion indicative of a user's intent to insert the manifold 30 into the receiver 26.

With further advancement of the manifold 30 into the receiver 26 and corresponding movement of the sled assembly 58 in the proximal direction, the cam 108 further rotates about the cam center axis CX. The point of contact between the roller 100 and the eccentric surface 116 moves from Point B towards Point A. The distance from Point B to the cam center axis CX is greater than the distance from Point A to the cam center axis CX. As a result, the lever biasing member 104 pivots the lever 98 correspondingly about the lever axis LX to maintain direct contact between the roller 100 and the cam 108. Less counterpart forces from the lever 98 are transmitted to the cam mechanism 96 and through the component stack up, which are felt as less resistance by the user handling the manifold 30. The reduced resistance may be desirable for the later stages to realize more of the mechanical advantages. In one implementation, the motion conversion assembly 64 may require a relatively short distance for which force is applied at the beginning and the end of insertion of the manifold 30, but otherwise provide for relatively free movement.

As mentioned, the movement of the motion conversion assembly 64 provides for movement of the inlet mechanism 32 in the distal direction, *i.e.*, the direction opposite to the movement of the sled assembly 58. In other words, the motion conversion assembly 64 converts motion of the sled assembly 58 into motion of the inlet mechanism 32. With the movement of the inlet mechanism 32 in the distal direction, the suction outlet 34 of the inlet mechanism 32 moves toward alignment with the receiver outlet 36. It is further realized that the motion conversion assembly 64 may provide an initial return movement of the manifold 30 after disengaging the lock assembly 48 from the manifold 30 via the actuator 46. The lock assembly 48 in the locked configuration engages the lock elements 82 of the manifold 30, and corresponding movement of the sled assembly 58 in the distal direction is prevented. Thus, the potential energy remains stored in the first biasing element 102. The actuator 46 being actuated moves the lock assembly 48 to the unlocked configuration as previously described, and movement of the sled assembly 58 in the distal direction is again permitted. When the user pushes or pulls the actuator 46 to disengage the arms 50, the potential energy stored in the first biasing element 102 is of sufficient magnitude to provide the initial return movement of the sled assembly 58 (and the manifold 30 coupled thereto) in the distal direction until the sled engagement pin 112 contact the proximal wall 111a of the slot 111. For example, stated simply, pressing the "ejector button" partially ejects the manifold 30 from the receiver 26 by a fixed amount. The partial ejection of the manifold 30 provides a visual indication to the user that the manifold 30 is no longer fully inserted into the receiver 26. The fixed amount by which the manifold 30 is partially ejected from the receiver 26 may be selectively tuned based on characteristics of the component stack up (*e.g.*, the spring constant of the sled biasing member 67 or the distance between the proximal and distal walls 111a, 11b). In some instances, the initial return movement is approximately one-quarter inch, but greater or lesser distances are contemplated. The fixed amount may be a small proportion of the length of the manifold 30, and should not be to an extent in which the manifold 30 may fully eject from the receiver 26 unexpectedly.

An electronics module (not shown) may be coupled to an upper wall of the housing 40. The electronics module may include any number of electronic subcomponents, for example, sensors, integrated circuits, printed circuit boards, memory, communications means, and electrical or data ports. For example, the electronics module may include one or more sensors for detecting positions of the sled assembly 58 of the receiver 26. A detectable element 120 may be positioned on the sled assembly 58.

The partial ejection of the manifold 30 may move the detectable element 120 coupled to the sled assembly 58 away from detectability from one or more sensors that may be coupled to the electronics module. For example, the one or more sensors may comprise a Hall effect sensor, and the detectable element 120 may comprise a magnet with alteration of the magnetic field being sensed by the Hall effect sensor. Alternative examples may include optical, electromagnetic, radiofrequency, and ultrasonic sensing of the detectable element. The electronics module may be in electronic communication with a system processor (not identified), and detection by the one or more sensors of an absence of the detectable element 120 may be indicative that the manifold 30 is not fully inserted (or no manifold is present). The initial return movement from the motion conversion assembly 64 may be of sufficient magnitude to space apart the detectable element 120 from the one or more sensors by a distance in which the one or more sensors generates a sled change signal. The sled change signal may be transmitted to the system processor, and any type of front-end functionality may be realized based on the sled change signal. For example, the medical waste collection system 20 may output a visual or audible warning to alert the user that the manifold 30 is not fully inserted. For another example, the medical waste collection system 20 may be electronically prevented from operation based on the sled change signal.

In another example, one or more other sensors may be coupled to the electronics module and configured to detect the detectable element coupled to the first barrier 44. For example, the sensor may be a Hall effect sensor, or any suitable optical, electromagnetic, radiofrequency, and ultrasonic sensor. Detection of the sensor of a presence of the detectable element is indicative that the first barrier 44 is in the open position. The sensor may generate and transmit a door change signal to the system processor, and any type of front-end functionality may be realized based on the door change signal. For example, the door change signal may be used in combination with the sled change signal in which it may be determined that the manifold 30 is partially but not fully inserted into the receiver 26 (*i.e.*, the first barrier 44 is open but the one or more sensors do not detect the detectable element 120).

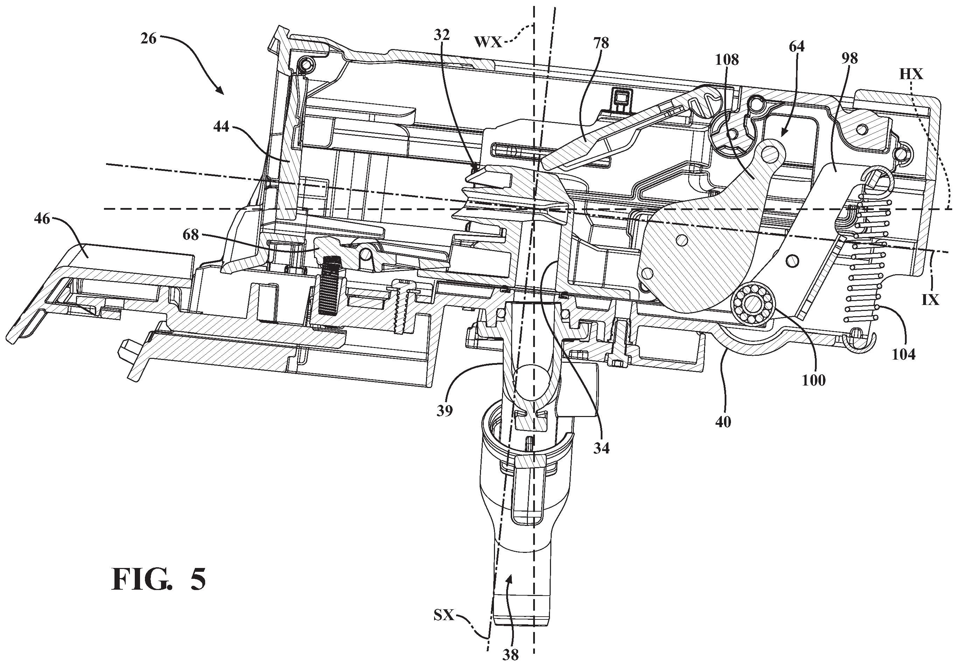

With reference to Figure 5, the suction outlet 34 is in fluid communication with the receiver outlet 36 and the conduit 38, and thus, the waste container 24. The inlet mechanism is moveable proximally along an inlet axis IX to break fluid coupling between the suction outlet 34 and the receiver outlet 36. The inlet axis IX may be disposed at a decline angle relative to a reference horizontal axis HX with respect to gravity. The decline angle may promote an advantageous angle of loading for the user and support excess fluid draining away from the opening 28. The conduit 38 may comprise a receiver coupling portion 39 extending along a conduit axis WX from the receiver 26 toward the waste container 24. The conduit axis WX may be oblique to the inlet axis IX. The conduit axis WX may be disposed vertically with respect to gravity to assist the packaging of the conduit 38 and the waste container 24 on the cart 22 and beneath the receiver 26. The suction outlet 34 may extend along a suction outlet axis SX that is oblique to the conduit axis. In some configurations, the suction outlet axis SX is perpendicular to the inlet axis IX.

A seal 80 may be coupled to the housing 40 to cover the receiver outlet 36. The seal 80 may be disposed between the housing 40 and the suction outlet 34 of the inlet mechanism 32. The seal 80 may include upper and lower surfaces that are angled relative to one another to provide the decline angle with the receiver coupling portion 39 oriented at a vertical angle. The upper and lower surfaces may be arranged at an angle within the range of two to seven degrees, and more particularly five degrees. The seal 80 may include a friction ring configured to maintain the seal despite friction from the inlet mechanism 32 repeatedly sliding along the upper surface of the seal 80. The friction ring may be at least partially formed from Teflon or other low friction material.

The foregoing description is not intended to be exhaustive or limit the invention to any particular form. The terminology which has been used is intended to be in the nature of words of description rather than of limitation. Many modifications and variations are possible in light of the above teachings and the invention may be practiced otherwise than as specifically described.

The following examples are also encompassed by the present disclosure and may fully or partly be incorporated into embodiments:
1. A medical waste collection system for collecting medical waste material through a manifold during a medical procedure, the medical waste collection system comprising:
   a waste container;
   a vacuum source configured to provide a vacuum on the waste container; and
   a receiver coupled to the waste container and comprising:
      a housing comprising an opening into which the manifold is configured to be inserted, the housing further comprising a receiver outlet;
      an inlet mechanism coupled to the housing so as to be moveable in proximal and distal directions along an inlet axis, wherein the inlet mechanism comprises a suction inlet, and a suction outlet in fluid communication with the suction inlet;
      a sled assembly moveably coupled to the housing and operably coupled to the inlet mechanism, wherein the sled assembly is configured to be moved in a proximal direction during insertion of the manifold into the receiver in the proximal direction to facilitate the inlet mechanism moving correspondingly in the distal direction to establish fluid communication between the suction outlet and the receiver outlet;
      a lock assembly coupled to the housing and configured to lock the manifold within the receiver in a fully inserted position; and
      an actuator coupled to the lock assembly and axially moveable relative to the housing, wherein the actuator is configured to receive an axial input from a user to cause the lock assembly to unlock the manifold.
2. The medical waste collection system of example 1, wherein the lock assembly comprises an arm rotatably coupled to the housing and an arm biasing member biasing the arm to a locked configuration where the arm is configured to abut the manifold in the fully inserted position to prevent distal movement of the manifold and the sled assembly.
3. The medical waste collection system of example 2, wherein the actuator comprises a ramped surface configured to abut the arm and rotate the arm in opposition to the biasing member away from the manifold to permit movement of the manifold and the sled assembly in the distal direction.
4. The medical waste collection system of any one of examples 2-3, wherein the sled assembly comprises a sled body configured to abut the manifold, the sled body movable to at least a proximal position when the manifold is in the fully inserted position and a distal position, and the sled body moveable with the manifold while the manifold is disposed within the opening of the receiver.
5. The medical waste collection system of example 4, wherein the sled assembly comprises a sled biasing member coupled to the sled body, the sled biasing member configured to bias the sled body distally against the arm while the arm is in the locked configuration, and wherein the sled biasing member is configured to move the sled body and the manifold distally from the proximal position and the fully inserted position, respectively, responsive to movement of the arm to an unlocked configuration.
6. The medical waste collection system of any one of examples 4-6, wherein the sled body comprises an arm retention surface configured to abut the arm of the lock assembly and retain the arm of the lock assembly in an unlocked configuration while the sled body is in the distal position.
7. A medical waste collection system for collecting medical waste material through a manifold during a medical procedure, the medical waste collection system comprising:
   a waste container;
   a vacuum source configured to provide a vacuum on the waste container; and
   a receiver coupled to the waste container and comprising:
      a housing comprising an opening into which the manifold is configured to be inserted, the housing further comprising a receiver outlet;
      an inlet mechanism coupled to the housing and comprising a suction inlet, and a suction outlet in fluid communication with the suction inlet, the inlet mechanism moveable between a first position where the suction outlet and the receiver outlet are not in fluid communication and a second position where the suction outlet and the receiver outlet are in fluid communication; and
      an inlet lock assembly having a latch configured to be moveably coupled to the housing, and a biasing member biasing the latch to a locked position in which movement of the inlet mechanism to the second position is prevented, wherein the latch is configured to be moveable responsive to abutting engagement with the manifold during insertion of the manifold in the receiver from the locked position to an unlocked position in which movement of the inlet mechanism to the second position is permitted.
8. The medical waste collection system of example 7, wherein the latch is pivotably coupled to the housing about a latch axis.
9. The medical waste collection system of example 8, wherein the latch comprises a head portion and a tail portion opposite the head portion from the latch axis, and optionally wherein the tail portion is longer than the head portion.
10. The medical waste collection system of any one of examples 7-8, wherein the inlet mechanism comprises an inlet base moveable between the first and second positions, and wherein the latch is configured to abut the inlet base in the locked position to prevent the inlet base from moving to the second position.
11. The medical waste collection system of example 10, wherein the inlet base defines a cavity for receiving the latch when the latch is in the unlocked position and the inlet base is in the second position.
12. The medical waste collection system of any one of examples 7-11, further comprising a sled assembly moveably coupled to the housing and operably coupled to the inlet mechanism, wherein the sled assembly is configured to be moved in a proximal direction during insertion of the manifold into the receiver in the proximal direction to facilitate the inlet mechanism moving correspondingly in a distal direction to the second position.
13. A medical waste collection system for collecting medical waste material through a manifold during a medical procedure, the medical waste collection system comprising:
   a waste container;
   a vacuum source configured to provide a vacuum on the waste container; and
   a receiver coupled to the waste container and comprising:
      a housing comprising an opening into which the manifold is configured to be inserted, the housing further comprising a receiver outlet;
      an inlet mechanism coupled to the housing so as to be moveable in proximal and distal directions, wherein the inlet mechanism comprises a suction inlet, and a suction outlet in fluid communication with the suction inlet;
      a sled assembly moveably coupled to the housing and operably coupled to the inlet mechanism, wherein the sled assembly is configured to be moved in a proximal direction during insertion of the manifold into the receiver in the proximal direction to facilitate the inlet mechanism moving correspondingly in the distal direction to establish fluid communication between the suction outlet and the receiver outlet; and
      a motion conversion assembly comprising a cam mechanism operatively coupling the sled assembly and the inlet mechanism to facilitate the respective corresponding movements of the sled assembly and the inlet mechanism in the proximal and distal directions.
14. The medical waste collection system of example 13, wherein the cam mechanism comprises a cam body rotatably coupled to the housing about a cam center axis, the cam body having an eccentric surface with a plurality of points of the eccentric surface being spaced from the cam center axis at different radial distances.
15. The medical waste collection system of example 14, wherein the inlet mechanism comprises an inlet base defining an inlet slot, and wherein the cam mechanism comprises an inlet mechanism engagement pin extending from the cam body and being received in the inlet slot, and wherein the inlet mechanism engagement pin is configured to move within the inlet slot and abut the inlet base to move the inlet mechanism proximally and distally in response to rotation of the cam body.
16. The medical waste collection system of any one of examples 14-15, wherein the sled assembly comprises a sled body defining a sled slot, and wherein the cam mechanism comprises a sled engagement pin extending from the cam body and being received in the sled slot, and wherein the sled engagement pin is configured to move within the sled slot and abut the sled body to move the sled assembly proximally and distally in response to rotation of the cam body.
17. The medical waste collection system of any one of examples 14-16, wherein the motion conversion assembly further comprises a cam follower mechanism configured to provide resistance to rotation of the cam body.
18. The medical waste collection system of example 17, wherein the cam follower mechanism comprises a lever rotatably coupled to the housing about a lever axis spaced from the cam center axis, and the cam follower mechanism comprising a roller rotatably coupled to the lever and configured to be in direct contact with the eccentric surface of the cam body.
19. The medical waste collection system of example 18, wherein the cam follower mechanism comprises a biasing element coupled to the lever to bias the roller into contact with the eccentric surface of the cam body and to provide resistance to rotation of the cam body.
20. The medical waste collection system of any one of examples 14-19, wherein the eccentric surface comprises a first point at a first radial distance from the cam center axis, a second point at a second radial distance from the cam center axis, and a third point at a third radial distance from the cam center axis, and wherein the second radial distance is greater than the first and third radial distances.
21. The medical waste collection system of example 20, wherein the second point is circumferentially disposed between the first and third points.
22. A medical waste collection system for collecting medical waste material through a manifold during a medical procedure, the medical waste collection system comprising:
   a waste container comprising a waste container inlet;
   a vacuum source configured to provide a vacuum on the waste container; and
   a receiver coupled to the waste container and comprising:
      a housing comprising an opening into which the manifold is configured to be inserted at a decline angle relative to horizontal, the housing further comprising a receiver outlet;
      an inlet mechanism coupled to the housing and comprising a suction inlet, and a suction outlet in fluid communication with the suction inlet, the inlet mechanism moveable along an inlet axis at the decline angle between a first position in which the suction outlet and the receiver outlet are not in fluid communication, and a second position in which the suction outlet and the receiver outlet are in fluid communication; and
      a conduit coupled to and extending between the receiver outlet and the waste container inlet to facilitate a transfer of waste material from the receiver outlet to the waste container, the conduit having a receiver coupling portion extending from the receiver outlet along a conduit axis that is oblique to the inlet axis.
23. The medical waste collection system of example 22, further comprising a seal coupled to the housing to cover the receiver outlet.
24. The medical waste collection system of any one of examples 22-23, wherein the suction outlet extends along a suction outlet axis that is oblique to the conduit axis.
25. The medical waste collection system of example 24, wherein the suction outlet axis is perpendicular to the inlet axis.
26. The medical waste collection system of any one of examples 22-25, further comprising a sled assembly moveably coupled to the housing and operably coupled to the inlet mechanism, wherein the sled assembly is configured to be moved in a proximal direction during insertion of the manifold into the receiver in the proximal direction to facilitate the inlet mechanism moving correspondingly in a distal direction to the second position.
27. The medical waste collection system of any one of examples 1-7, 12, 13-21, and 26, wherein the sled assembly is configured to be moved in a distal direction opposite the proximal direction during removal of the manifold from the receiver to facilitate the inlet mechanism moving correspondingly in the proximal direction to break fluid communication between the suction outlet and the receiver outlet.
28. The medical waste collection system of example 27, wherein the receiver further comprises a claw coupled to the sled assembly, wherein the claw is configured to selectively engage the manifold and facilitates movement of the sled assembly in the distal direction during removal of the manifold from the receiver.
29. The medical waste collection system of any one of examples 1-7, 12, 13-21, and 26-28, further comprising an electronics module in communication with the vacuum source, wherein the receiver further comprises a sensor in communication with the electronics module and configured to output a signal indicative of a position of the sled assembly in the proximal and distal directions, wherein the electronics module is configured to control the vacuum source based on the signal from the sensor.
30. The medical waste collection system of example 29, further comprising a magnet disposed on the sled assembly and configured to be detected by the sensor.
31. The medical waste collection system of example 29, wherein the signal is indicative of whether the manifold is inserted into the receiver to a fully inserted position, wherein the electronics module is configured to prevent operation of the vacuum source based on the signal from the sensor when the manifold is not inserted into the receiver to the fully inserted position.
32. The medical waste collection system of any one of examples 1-7, 12, 13-21, and 26-31, wherein the receiver further comprises a first barrier pivotably coupled to the housing, and a first biasing element coupled to the first barrier configured to bias the first barrier towards a closed position to selectively cover at least a portion of the opening of the receiver.
33. The medical waste collection system of example 32, wherein the receiver further comprises a second barrier pivotably coupled to the sled assembly and positioned proximal to the first barrier, and a second biasing element coupled to the second barrier configured to bias the second barrier towards a closed position.
34. The medical waste collection system of example 33, wherein movement of the inlet mechanism in the distal direction facilitates moving the second barrier from the closed position to an open position in which the suction inlet of the inlet mechanism is exposed to the manifold being inserted.

## Claims

1. A medical waste collection system (20) for collecting medical waste material through a manifold (30) during a medical procedure, the medical waste collection system (20) comprising:
a waste container (24);
a vacuum source configured to provide a vacuum on the waste container (24); and
a receiver (26) coupled to the waste container (24) and comprising:
a housing (40) comprising an opening (28) into which the manifold (30) is configured to be inserted, the housing (40) further comprising a receiver outlet (36);
an inlet mechanism (32) coupled to the housing (40) so as to be moveable in proximal and distal directions, wherein the inlet mechanism (32) comprises a suction inlet (33), and a suction outlet (34) in fluid communication with the suction inlet (33);
a sled assembly (58) moveably coupled to the housing (40) and operably coupled to the inlet mechanism (32), wherein the sled assembly (58) is configured to be moved in a proximal direction during insertion of the manifold (30) into the receiver (26) in the proximal direction to facilitate the inlet mechanism (32) moving correspondingly in the distal direction to establish fluid communication between the suction outlet (34) and the receiver (26) outlet; and
a motion conversion assembly (64) comprising a cam mechanism (96) operatively coupling the sled assembly (58) and the inlet mechanism (32) to facilitate the respective corresponding movements of the sled assembly (58) and the inlet mechanism (32) in the proximal and distal directions.

2. The medical waste collection system (20) of claim 1, wherein the cam mechanism (96) comprises a cam body (108) rotatably coupled to the housing (40) about a cam center axis (CX), the cam body (108) having an eccentric surface (116) with a plurality of points (A, B, C) of the eccentric surface (116) being spaced from the cam center axis (CX) at different radial distances.

3. The medical waste collection system (20) of claim 2, wherein the inlet mechanism (32) comprises an inlet base (69) defining an inlet slot (105), and wherein the cam mechanism (96) comprises an inlet mechanism (32) engagement pin extending from the cam body (108) and being received in the inlet slot (105), and wherein the inlet mechanism (32) engagement pin is configured to move within the inlet slot (105) and abut the inlet base (69) to move the inlet mechanism (32) proximally and distally in response to rotation of the cam body (108).

4. The medical waste collection system (20) of one of claim 2 and claim 3, wherein the sled assembly (58) comprises a sled body (59) defining a sled slot (111), and wherein the cam mechanism (96) comprises a sled engagement pin (112) extending from the cam body (108) and being received in the sled slot (111), and wherein the sled engagement pin (112) is configured to move within the sled slot (111) and abut the sled body (59) to move the sled assembly (58) proximally and distally in response to rotation of the cam body (108).

5. The medical waste collection system (20) of any one of claims 2-4, wherein the motion conversion assembly (64) further comprises a cam follower mechanism (97) configured to provide resistance to rotation of the cam body (108).

6. The medical waste collection system (20) of claim 5, wherein the cam follower mechanism (97) comprises a lever (98) rotatably coupled to the housing (40) about a lever axis (LX) spaced from the cam center axis (CX), and the cam follower mechanism (97) comprising a roller (100) rotatably coupled to the lever (98) and configured to be in direct contact with the eccentric surface (116) of the cam body (108).

7. The medical waste collection system (20) of claim 6, wherein the cam follower mechanism (97) comprises a biasing element (104) coupled to the lever (98) to bias the roller (100) into contact with the eccentric surface (116) of the cam body (108) and to provide resistance to rotation of the cam body (108).

8. The medical waste collection system (20) of any one of claims 2-7, wherein the eccentric surface (116) comprises a first point (C) at a first radial distance from the cam center axis (CX), a second point (B) at a second radial distance from the cam center axis (CX), and a third point (A) at a third radial distance from the cam center axis (CX), and wherein the second radial distance is greater than the first and third radial distances, and wherein, optionally, the second point is circumferentially disposed between the first and third points.

9. The medical waste collection system (20) of any one of claims 1-8, wherein the sled assembly (58) is configured to be moved in a distal direction opposite the proximal direction during removal of the manifold (30) from the receiver (26) to facilitate the inlet mechanism (32) moving correspondingly in the proximal direction to break fluid communication between the suction outlet (34) and the receiver (26) outlet.

10. The medical waste collection system (20) of claim 9, wherein the receiver (26) further comprises a claw (62) coupled to the sled assembly (58), wherein the claw (62) is configured to selectively engage the manifold (30) and facilitates movement of the sled assembly (58) in the distal direction during removal of the manifold (30) from the receiver (26).

11. The medical waste collection system (20) of any one of claims 1-10, further comprising an electronics module in communication with the vacuum source, wherein the receiver (26) further comprises a sensor in communication with the electronics module and configured to output a signal indicative of a position of the sled assembly (58) in the proximal and distal directions, wherein the electronics module is configured to control the vacuum source based on the signal from the sensor.

12. The medical waste collection system (20) of claim 11, further comprising a magnet (120) disposed on the sled assembly (58) and configured to be detected by the sensor.

13. The medical waste collection system (20) of claim 11, wherein the signal is indicative of whether the manifold (30) is inserted into the receiver (26) to a fully inserted position, wherein the electronics module is configured to prevent operation of the vacuum source based on the signal from the sensor when the manifold (30) is not inserted into the receiver (26) to the fully inserted position.

14. The medical waste collection system (20) of any one of claims 1-10, wherein the receiver (26) further comprises a first barrier (44) pivotably coupled to the housing (40), and a first biasing element coupled to the first barrier (44) configured to bias the first barrier (44) towards a closed position to selectively cover at least a portion of the opening (28) of the receiver (26).

15. The medical waste collection system (20) of claim 14, wherein the receiver (26) further comprises a second barrier (78) pivotably coupled to the sled assembly (58) and positioned proximal to the first barrier (44), and a second biasing element coupled to the second barrier (78) configured to bias the second barrier (78) towards a closed position, and wherein, optionally, movement of the inlet mechanism (32) in the distal direction facilitates moving the second barrier (78) from the closed position to an open position in which the suction inlet (33) of the inlet mechanism (32) is exposed to the manifold (30) being inserted.
